# EUROPÄISCHE PATENTSCHRIFT

(11) **EP 1 681 028 B1**
(45) Veröffentlichungstag und Bekanntmachung des Hinweises auf die Patenterteilung: **26.03.2014**
(21) Anmeldenummer: 05000467.0
(22) Anmeldetag: 12.01.2005
(51) Int. Cl.: A61B 19/00

(54) **Video-tracking und -Registrierung**
Video tracking and registration
Système et procédé de poursuite et d'enregistrement vidéo

(43) Veröffentlichungstag der Anmeldung: 19.07.2006
(73) Patentinhaber: Brainlab AG, 85622 Feldkirchen (DE)
(72) Erfinder: Manus, Johannes, Dr., 81479 München (DE)
(74) Vertreter: Schwabe - Sandmair - Marx

(56) Entgegenhaltungen:
- EP-A- 1 319 368
- WO-A1-01/74266
- WO-A1-2004/107157
- US-A- 6 006 126
- US-A1- 2002 002 330
- US-A1- 2002 099 293
- US-A1- 2002 183 608
- US-A1- 2004 068 187

## Beschreibung

Die vorliegende Erfindung betrifft ein Tracking- und Registrierungsverfahren sowie eine Vorrichtung zum räumlichen Orten und Verfolgen eines medizinischen Instruments und eines Patienten bzw. eines Patientenkörperteils, wobei mittels einer Kameraanordnung mit mindestens zwei Kameras Aufnahmen des Instruments und bzw. des Patientenkörperteils des Patienten gemacht werden, womit das Instrument oder der Patient registriert und getrackt werden.

Bekannte medizinische Navigationssysteme verwenden in der Regel ein Tracking-System zum Verfolgen eines Körpers eines Patienten oder eines Instrumentes, wie einem Registrierungsinstrument, welches wiederum, z.B. durch Abtasten der Oberfläche eines Patienten einen Registrierungsvorgang gewährleistet. Da mehrere Geräte zum Registrieren und Tracken verwendet werden, kommt es häufig zu Ungenauigkeiten und daraus resultierenden Berechnungsfehlern, die sich insbesondere über den gesamten Trackingvorgang fortpflanzen.

Aus der US 6,006,126 ist ein Verfahren bekannt, bei welchem mittels einer Kameraanordnung ein Patient und medizinische Instrumente unter Zuhilfenahme von Trackingmarker verfolgt werden. Anhand der Trackingmarker auf dem Patienten werden sowohl die mittels der einzelnen Kameras der Kameraanordnung erstellten Bilder einander zugeordnet, um die dreidimensionalen Koordinaten der auf den Bildern zu sehenden Trackingmarker zu erhalten, als auch das mittels der Kameraanordnung erstellte Realbild des Patienten einem zuvor erstellten Bilddatensatz des Patienten im dreidimensionalen Raum.

Es ist eine Aufgabe der vorliegenden Erfindung eine Vorrichtung und ein Verfahren vorzuschlagen, welche kostengünstige und genaue Tracking- und Registrierungsvorgänge ermöglichen.

Das erfindungsgemäße Verfahren ist ein Tracking- und Registrierungsverfahren gemäß dem Anspruch 1. Die Unteränsprüche definieren bevorzugte Ausführungsformen der Erfindung. Der akquirierte, gespeicherte Patientendatensatz kann mittels eines bildgebenden Verfahrens ermittelt werden. Als bildgebendes Verfahren zur Ermittlung des Patientendatensatzes kann z.B. ein Röntgen-Verfahren, ein Kernspin-Verfahren, ein Computertomographie-Verfahren, ein Ultraschall-Verfähren, ein Positronen-Emissions-Tomographie-Verfahren (PET-Verfahren) oder ein Single Photon Emission Tomography-Verfahren (SPECT-Verfahren) verwendet werden. Mit der Kameraanordnung werden Trackingmarkierungen, wie Marker oder Referenzsterne, welche auf dem medizinischen Instrument und auf dem Patienten bzw. am Körperteil angebracht sind, aufgenommen. Die Marker oder die Referenzsterne können aktive, wie z.B. Infrarot-Strahlung oder sichtbares Licht emittierende Markierungen sein, oder können passive, wie Infrarot-Strahlung oder sichtbares Licht reflektierende Markierungen sein. Die aufgenommenen Trackingmarkierungen werden aus den Kameraaufnahmen extrahiert und verfolgt, um die Ortung und Verfolgung des medizinischen Instruments und des Patienten bzw. des Körperteils des Patienten zu gewährleisten oder durchzuführen. Der Abstand oder die Position, wie die Ausgangsposition, der Trackingmarkierungen, die auf dem medizinischen Instrument und dem Patienten bzw. dem Patientenkörperteil aufgebracht sind, zu der Kameraanordnung kann bekannt sein oder kann z.B. mittels eines Kalibrierungsvorgangs festgelegt oder ermittelt werden, so dass die Lage der Trackingmarkierungen im Raum durch die Erfassung mittels der Kameraanordnung bekannt sein kann oder ermittelt werden kann oder Lageveränderungen mittels der Kameraanordnung erfasst und verfolgt werden.

Aus denselben oder weiteren mittels derselben Kameraanordnung gemachten Videoaufnahmen der Oberfläche des Patienten bzw. des Körperteils werden Oberflächenteile identifiziert, wie natürliche oder künstliche, z.B. künstlich aufgebrachte Landmarken, welche computergestützt mit entsprechenden Oberflächenteilen in dem gespeicherten Patientendatensatz in Korrespondenz gebracht werden, um die Zuordnung der Raumposition des Patienten bzw. des Körperteils des Patienten und des Patientendatensatzes durchzuführen. Insbesondere können die Aufnahmen gleichzeitig mittels der mindestens zwei Kameras, welche bezüglich eines gemeinsamen Koordinatensystems, wie dem Tracking-System-Koordinatensystem, kalibriert sein können, durchgeführt werden. Die Aufnahmen, die vorzugsweise aus unterschiedlichen Positionen der Kameras erfolgt sind, werden für den Registrierungsvorgang verwendet. Der Registrierungsvorgang wird über die Identifizierung der Oberflächenteile in den Videoaufnahmen durchgeführt, welche mit den korrespondierenden Oberflächenteilen in dem gespeicherten Patientendatensatz in Korrespondenz gebracht werden, um eine Registrierung zu erreichen. Da die Videoaufnahmen bereits aus einer bekannten Position relativ zu dem verwendeten Koordinatensystem aufgenommen werden, wie z.B. dem Ursprung des Tracking-System-Koordinatensystems oder des Kamera-Koordinatensystems, sind zusätzliche Ortsinformationen über die Kameraanordnung oder die mindestens zwei Kameras für einen Registrierungsvorgang nicht zwingend notwendig.

Vorzugsweise ist der Abstand der mindestens zwei Kameras der Kameraanordnung zueinander bekannt oder er kann z.B. mittels eines Kalibrierungsvorgangs bestimmt werden. Mit den Kameras können z.B. jeweils ein Bild oder mehrere Bilder aufgenommen werden und es können charakteristische Punkte oder Oberflächenteile in den Aufnahmen identifiziert werden, deren Raumpositionen, wie deren Positionen im Weltkoordinatensystem, z.B. ermittelt werden können, da die Position der Kameras zueinander oder bezüglich des Weltkoordinatensystems, wie dem Tracking-System-Koordinatensystem oder dem Kameraanordnung-Koordinatensystem, bekannt ist oder bestimmt werden kann. Dies ist vorteilhaft gegenüber Systemen, bei denen die Positionen der Kameras z.B. während der Aufnahmevorgänge von einem Tracking-System verfolgt werden, da im Allgemeinen derartige Positionsbestimmungen ungenauer sind als eine Positions- oder Lagebestimmung der mindestens zwei Kameras bezüglich der Kameraanordnung mittels eines Kalibrierungsvorganges.

Vorzugsweise können als Oberflächenteile des Patienten bzw. des Patientenkörperteils, welche in den gemachten Videoaufnahmen identifiziert werden können, insbesondere korrespondierende Punkte, Punktmengen, Punkttupel oder Konturen der Oberfläche verwendet werden. Dabei werden die identifizierten Oberflächenteile z.B. mittels eines computergestützten Matchingverfahrens mit den entsprechenden Oberflächenteilen des Patientendatensatzes in Korrespondenz gebracht. Die Oberflächenteile der gemachten Aufnahmen, können z.B. natürliche Landmarken oder künstliche, z.B. künstlich aufprojezierte, aufgezeichnete oder angebrachte Landmarkenpunkte sein. Auch werden die Oberflächenteile mittels eines computergestützten Morphingverfahrens oder Warpingverfahrens ermittelt wie es z.B. in der EP 1 667 678 A1 beschrieben wird (Stand der Technik gem. Art. 54 (3) EPÜ)**.**

Der z.B. mittels eines bildgebenden Verfahrens akquirierte Patientendatensatz und die gemachten Videoaufnahmen bzw. Kameraaufnahmen, insbesondere die identifizierten Oberflächenteile, können z.B. in einem medizinischen Navigationssystem, dem die Tracking- und Registrierungs-Kameraanordnung zugeordnet sein kann, verarbeitet oder gespeichert werden. Auch wird in dem Computer des Navigationssystems die Korrespondenz zwischen den identifizierten Oberflächenteilen und dem Patientendatensatz, nämlich den korrespondierenden Oberflächenteilen des Patientendatensatzes, durchgeführt, so dass in dem Navigationssystem eine Registrierung der gemachten Aufnahmen bezüglich des gespeicherten Patientendatensatzes erfolgt.

Die Aufnahmen für das Tracken bzw. die Ortung und für die Raumpositions-Patientendatensatz-Zuordnung können z.B. im sichtbaren Lichtbereich erstellt werden bzw. mittels sichtbaren Lichts erfolgen. Auch können die Aufnahmen für das Tracken bzw. die Ortung im Infrarot-Bereich erstellt werden bzw. mittels Infrarot-Strahlung erstellt werden, indem z.B. die Infrarot-Strahlung der aktiven oder passiven Trackingmarkierungen erfasst wird, während die Aufnahmen für die Raumpositions-Patientendatensatz-Zuordnung z.B. im sichtbaren Lichtbereich aufgenommen werden.

Die Erfindung bezieht sich des Weiteren auf ein Computerprogramm, welches, wenn es in einen Computer geladen wird oder auf einem Computer läuft, ein wie oben beschriebenes Verfahren durchführt. Weiterhin bezieht sich die Erfindung auf ein Programmspeichermedium oder ein Computerprogrammprodukt mit einem solchen Programm.

Die erfindungsgemäße Vorrichtung ist im Anspruch 10 definiert.

Die mindestens zwei Kameras der Kameraanordnung können insbesondere für Videoaufnahmen im Bereich des sichtbaren Lichts ausgebildet sein, so dass sie sichtbares Licht emittierende oder reflektierende Objekte, wie den Patienten bzw. das Körperteil des Patienten und die medizinischen Instrumente aufnehmen können. Die mindestens zwei Kameras können auch als Hybrid-Kameras ausgebildet sein, die sowohl Videoaufnahmen im Bereich des sichtbaren Lichts als auch im Infrarot-Bereich durchführen können, um z.B. Infrarot-Strahlung reflektierende oder emittierende Trackingmarkierungen, wie Marker oder Referenzsterne, erfassen zu können.

Insbesondere kann die Kameraanordnung fest oder beweglich ausgebildet sein, um Aufnahmen des Patienten oder des Instruments durchzuführen.

Auch kann die erfindungsgemäße Vorrichtung eine Eingabevorrichtung, wie eine Tastatur, eine Röntgenvorrichtung, einen Ultraschall-Tomographen, eine Magnetresonanz-Tomographen, einen Computer-Tomographen, einen Positronen-Emissions-Tomographen oder einen großen SPECT-Tomographen umfassen, mit welcher z.B. vor oder während einer Operation der Körper bzw. das Körperteil eines Patienten oder ein medizinisches Instrument erfasst und z.B. in das Navigationssystem als Referenzpatientendatensatz eingelesen werden kann, bezüglich dessen der Registrierungsvorgang durchgeführt werden kann. Des Weiteren kann die Vorrichtung auch eine Datenbank umfassen, in welche die erfassten Patientendaten eingelesen und gespeichert werden können. Das Navigationssystem kann z.B. auf die Datenbank zugreifen und einen gewünschten Patientendatensatz als Referenzdatensatz für die Registrierung auswählen. Auch kann die erfindungsgemäße Vorrichtung eine Datenausgabevorrichtung, wie einen Bildschirm aufweisen, auf welchem z.B. der Registrierungsvorgang oder der Trackingvorgang oder der Navigationsvorgang grafisch dargestellt werden kann, um z.B. die Navigation eines Instruments bezüglich des zu untersuchenden Körpers zu ermöglichen.

Die Erfindung wird nachfolgend anhand bevorzugter Ausführungsbeispiele beschrieben werden. Es zeigen:
- Figur 1: Teile einer Vorrichtung gemäß einer Ausführungsform der vorliegenden Erfindung;
- Figur 2: eine Vorrichtung gemäß einer Ausführungsform der vorliegenden Erfindung.

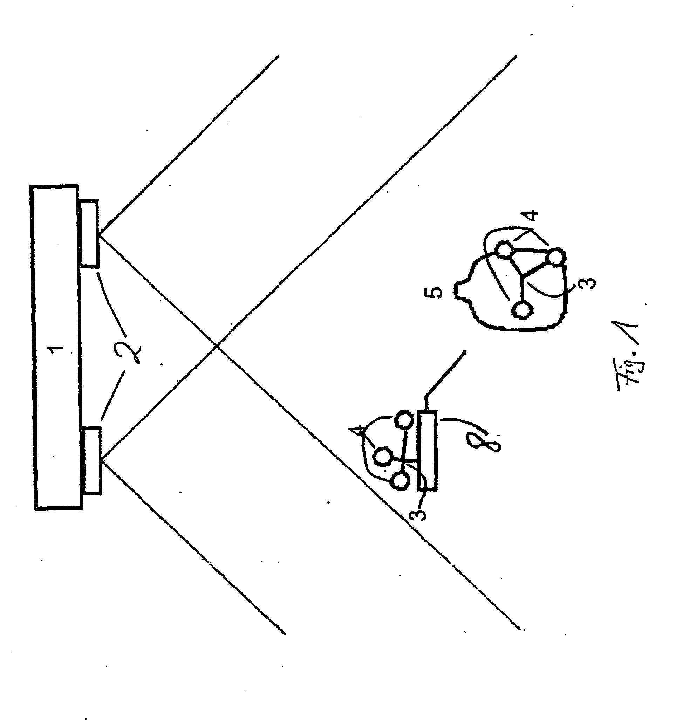

Figur 1 zeigt Teile einer Ausführungsform der vorliegenden Erfindung mit einer Kameraanordnung 1, welche zwei Kameras 2, insbesondere Hybrid-Kameras aufweist, die sowohl Aufnahmen im Bereich des sichtbaren Lichts als auch im Infrarot-Bereich durchführen können. Auch können anstelle der Hybrid-Kameras Kameras 2 verwendet werden, die im sichtbaren Lichtbereich arbeiten. Des Weiteren zeigt Figur 1 den Körper 5 eines Patienten, auf welchem ein Referenzstern 3 mit Markern 4, insbesondere mit passiven reflektierenden Markern 4, angeordnet ist, und ein Instrument 8, auf welchem ein Referenzstern 3 mit Markern 4 angebracht ist, mittels dessen z.B. eine Untersuchung des Körpers 5 durchgeführt wird. Der Körper 5 und das Instrument 8 befinden sich im Aufnahmebereich beider Kameras 2, sodass eine Aufnahme des Körpers 5 und des Instruments 8 aus verschiedenen Positionen erfolgen kann, wobei mittels der aus unterschiedlichen Positionen gemachten Aufnahmen ein Registrierungsvorgang bezüglich eines gemeinsamen Koordinatensystems, insbesondere des Koordinatensystems der Kameraanordnung 1, durchgeführt wird. Insbesondere wird mit derselben Kameraanordnung 1 bzw. mit den Kameras 2 die räumliche Position des Instruments 8 und des Körpers 5 erfasst und verfolgt, indem z.B. die Ausgangsposition des Instruments 8 und des Körpers 5 mittels eines Kalibrierungsvorganges bestimmt wird, die Kameras 2 die Referenzsterne 3 des Instruments 8 sowie des Körpers 5 erfassen und aus Veränderungen der Position der Referenzsterne auf Bewegungen des Instruments 8 und des Körpers 5 geschlossen werden kann. Somit wird mittels derselben Kameraanordnung 1 bzw. den an derselben Kameraanordnung 1 angebrachten Kameras 2 ein Registrierungsvorgang des Instruments 8 und des Körpers 5, insbesondere bezüglich eines gemeinsamen Koordinatensystems wie des Koordinatensystems der Kameraanordnung 1, durchgeführt, indem das Instrument 8 und der Körper 5 im sichtbaren Lichtbereich von den Kameras 2 erfasst wird und die erfassten Daten räumlichen Positionen, insbesondere Positionen bezüglich des gemeinsamen Koordinatensystems, zugeordnet werden. Auch wird mit derselben Kameraanordnung 1 ein Trackingvorgang des Instruments 8 und des Körpers 5 durchgeführt, indem z.B. die Referenzsterne 3 des Instruments 8 und des Körpers 5, welche z.B. von den Kameras 2 ausgesandte Infrarot-Strahlung reflektieren oder selbst Infrarot-Strahlung emittieren, von den Kameras 2 erfasst werden und deren Positionsveränderungen verfolgt werden.

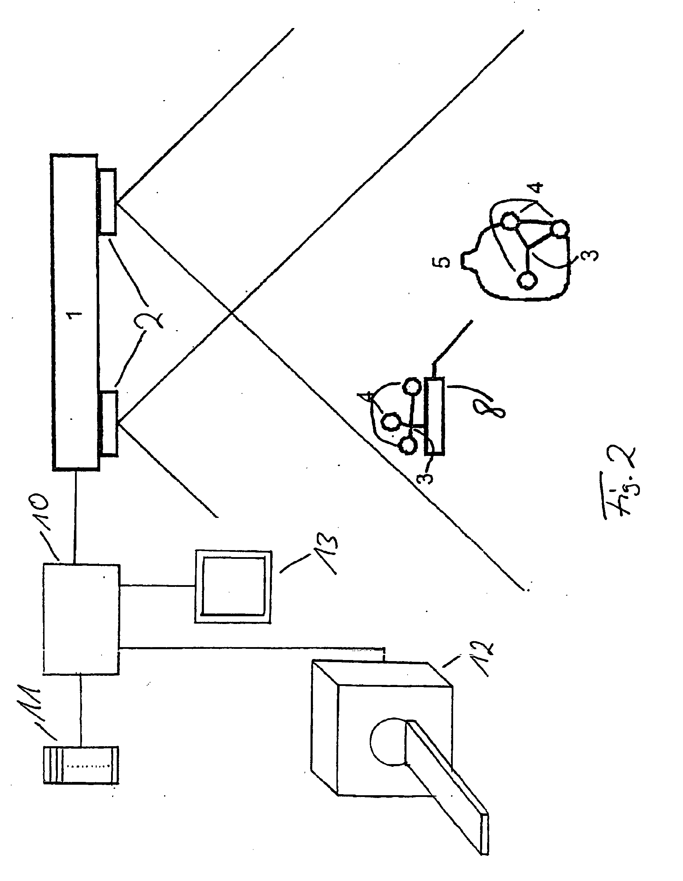

Figur 2 zeigt eine Ausführungsform der vorliegenden Erfindung mit einer Kameraanordnung 1, an der zwei Kameras 2 angebracht sind, wobei die Kameraanordnung 1 mit einem Navigationssystem 10, insbesondere einem Computer, drahtlos oder drahtgebunden verbunden ist. Die von den Kameras 2 erfassten Daten zum Tracken oder Registrieren des Instruments 8 und des Körpers 5 werden von der Kameraanordnung 1 an das Navigationssystem 10 übertragen werden, welches die Daten z.B. auswertet oder weiterverarbeitet und in einer Datenbank 11 speichert oder auf einer Datenausgabevorrichtung 13, wie einem Bildschirm, ausgibt. Auch können mit einer Dateneingabevorrichtung 12, die in der vorliegenden Ausführungsform als Computertomograph ausgebildet ist, Daten über einen Körper oder ein Instrument, wie den Körper 5 oder das Instrument 8, erfasst werden und an das Navigationssystem 10 gesendet werden, welches die erfassten Daten z.B. für einen Registrierungsvorgang, insbesondere mit den mittels den Kameras 2 erfassten Daten, verwenden kann oder die mittels der Dateneingabevorrichtung 12 erfassten Daten in der Datenbank 11 abspeichern kann, so dass die Daten in der Datenbank 11 z.B. als Referenzdaten gespeichert sein können und von dem Navigationssystem 10 ausgelesen werden können.

## Patentansprüche

1. Tracking- und Registrierungsverfahren zum räumlichen Orten und Verfolgen eines medizinischen Instruments (8) und eines Patienten (5) bzw. eines Patientenkörperteils mit einer Kameraanordnung (1), die mindestens zwei Kameras (2) aufweist, sowie zum gegenseitigen Zuordnen der Raumposition des Patienten (5) bzw. des Körperteils des Patienten (5) und eines akquirierten, gespeicherten Patientendatensatzes, bei dem mittels derselben Kameraanordnung (1)
- Trackingmarkierungen (4) auf dem medizinischen Instrument (8) und dem Patienten (5) bzw. dem Patientenkörperteil aufgenommen, aus den Kameraaufnahmen extrahiert und verfolgt werden, um die Ortung und die Verfolgung des medizinischen Instruments (8) und des Patienten (5) bzw. des Körperteils durchzuführen; und
- Videoaufnahmen der Oberfläche des Patienten (5) bzw. des Körperteils erstellt werden, in denen Oberflächenteile unterstützt durch ein computergestütztes Morphingverfahren identifiziert werden, welche durch ein computergestütztes Matchingverfahren mit entsprechenden Oberflächenteilen in dem gespeicherten Patientendatensatz in Korrespondenz gebracht werden, um die Zuordnung der Raumposition des Patienten (5) bzw. des Körperteils eines Patienten (5) und des Patientendatensatzes durchzuführen.

2. Verfahren nach Anspruch 1, bei dem als identifizierte Oberflächenteile Punkte, Punktemengen, Punkttupel oder Konturen der Oberfläche verwendet werden.

3. Verfahren nach einem der Ansprüche 1 bis 2, wobei der gespeicherte Patientendatensatz mit den Oberflächenteilen mittels eines Photokonsistenzverfahrens in Korrespondenz gebracht wird.

4. Verfahren nach einem der Ansprüche 1 bis 3, wobei der gespeicherte Patientendatensatz mit den Oberflächenteilen mittels eines Punktkorrelationsverfahrens in Korrespondenz gebracht wird.

5. Verfahren nach einem der Ansprüche 1 bis 4, bei dem die Speicherung des Patientendatensatzes und der identifizierten Oberflächenteile, sowie die Herstellung der Korrespondenz computergestützt mittels eines medizinischen Navigationssystems durchgeführt werden, dem die Kameraanordnung (1) zugeordnet ist.

6. Verfahren nach einem der Anspruche 1 bis 5, bei dem die Kameras für die Ortung und Verfolgung und für die Raumpositions-/Patientendatensatz-Zuordnung Videoaufnahmen im sichtbaren Lichtbereich erstellen.

7. Verfahren nach einem der Anspruche 1 bis 6, bei dem die Kameras für die Ortung und Verfolgung Aufnahmen im Infrarotlichtbereich erstellen und für die Raumpositions-/Patientendatensatz-Zuordnung Videoaufnahmen im sichtbaren Lichtbereich aufnehmen.

8. Programm, das, wenn es auf einem Computer läuft oder in einem Computer geladen ist, den Computer veranlasst, ein Verfahren gemäß einem der Ansprüche 1 bis 7 durchzuführen.

9. Computerprogramm-Speichermedium, das ein Programm nach Anspruch 8 aufweist.

10. Vorrichtung zum räumlichen Orten und Verfolgen eines medizinischen Instruments (8) und eines Patienten (5) bzw. eines Patientenkörperteils, die einen Computer (10) und eine einzige mit dem Computer (10) verbundene Kameraanordnung (1) mit mindestens zwei Kameras (2) aufweist, wobei die Kameraanordnung (1) ausgestaltet ist, Trackingmarkierungen auf dem medizinischen Instrument (8) und dem Patienten (5) bzw. dem Patientenkörperteil aufzunehmen, wobei der Computer (10) mittels eines darauf geladenen Programms ausgestaltet ist, die Trackingmarkierungen aus den Kameraaufnahmen zu extrahieren und zu verfolgen, um die Ortung und die Verfolgung des medizinischen Instruments (8) und des Patienten (5) bzw. des Körperteils durchzuführen, wobei die Kameraanordnung (1) ferner dazu ausgestaltet ist, Videoaufnahmen der Oberfläche des Patienten (5) bzw. des Körperteils zu erstellen, wobei der Computer (10) mittels des Programms ferner dazu ausgestaltet ist, in den Videoaufnahmen Oberflächenteile mittels eines Morphingverfahrens zu identifizieren und mittels eines Matchingverfahrens mit entsprechenden Oberflächenteilen in dem gespeicherten Patientendatensatz in Korrespondenz zu bringen, um die Zuordnung der Raumposition des Patienten (5) bzw. des Körperteils des Patienten (5) und des Patientendatensatzes durchzuführen.

11. Vorrichtung nach Anspruch 10, wobei beide Kameras (2) als Videokameras für Videoaufnahmen im Bereich des sichtbaren Lichts ausgebildet sind.

12. Vorrichtung nach Anspruch 10 oder 11, wobei beide Kameras (2) als Hybrid-Kameras für Videoaufnahmen im Bereich des sichtbaren Lichts und im Infrarot-Bereich ausgebildet sind.

13. Vorrichtung nach einem der Ansprüche 10 bis 12, wobei die Kameraanordnung (1) fest oder beweglich ausgebildet ist.

14. Vorrichtung nach einem der Ansprüche 10 -bis 13, wobei die Vorrichtung eine Datenausgabevorrichtung (13), wie einen Bildschirm zur Ausgabe des gespeicherten Patientendatensatzes und/oder des Patienten (5) bzw. des Patientenkörperteils und/oder des medizinischen Instrumentes (8) umfasst und/oder eine Dateneingabevorrichtung (12), insbesondere eine Tastatur, eine Röntgenvorrichtung, einen Ultraschall-Tomographen, einen Computer-Tomographen, einen Magenetresonanz-Tomographen, einen Positronen-Emissions-Tomographen (PET) oder einen Single-Photon-Emission-Tomography-Tomographen (SPET) umfasst, mittels welcher Daten über das medizinische Instrument (8) und/oder den Patienten (5) bzw. das Patientenkörperteil gewonnen werden können.

15. Vorrichtung nach einem der Ansprüche 10 bis 14, wobei die Vorrichtung eine Datenbank (11) umfasst, in welcher charakteristische Patientendatensätze als Referenzdatensätze gespeichert sein können oder in welche Patientendatensätze, insbesondere mittels der Dateneingabevorrichtung (12) als Referenzdatensätze eingelesen und gespeichert werden können.

16. Vorrichtung nach einem der Ansprüche 10 bis 15, wobei die Vorrichtung in einem Instrument, wie einem Mikroskop oder einem Endoskop, ausgebildet ist.

## Claims

1. A tracking and registering method for spatially locating and tracking a medical instrument (8) and a patient (5) or part of a patient's body using a camera array (1) comprising at least two cameras (2), and for mutually assigning the spatial position of the patient (5) or part of the body of the patient (5) and an acquired, stored patient data set, wherein by means of the same camera array (1):
- tracking markings (4) on the medical instrument (8) and the patient (5) or part of the patient's body are recorded, extracted from the camera recordings and tracked, in order to locate and track the medical instrument (8) and the patient (5) or part of the body; and
- video recordings of the surface of the patient (5) or part of the body are produced, in which surface portions are identified with the assistance of a computer-assisted morphing method and correlated by a computer-assisted matching method with corresponding surface portions in the stored patient data set, in order to assign the spatial position of the patient (5) or part of the body of a patient (5) and that of the patient data set.

2. The method according to claim 1, wherein points, point sets, tuples of points or contours of the surface are used as identified surface portions.

3. The method according to any one of claims 1 to 2, wherein the stored patient data set is correlated with the surface portions by means of a photo consistency method.

4. The method according to any one of claims 1 to 3, wherein the stored patient data set is correlated with the surface portions by means of a point correlation method.

5. The method according to any one of claims 1 to 4, wherein the patient data set and the identified surface portions are stored and correlated with computer assistance by means of a medical navigation system to which the camera array (1) is assigned.

6. The method according to any one of claims 1 to 5, wherein the cameras produce video recordings in the visible light range, for locating and tracking and for assigning the spatial positions and the patient data set.

7. The method according to any one of claims 1 to 6, wherein the cameras produce recordings in the infrared light range for locating and tracking and record video recordings in the visible light range for assigning the spatial positions and the patient data set.

8. A program which, when it is running on a computer or is loaded onto a computer, causes the computer to perform a method in accordance with any one of claims 1 to 7.

9. A computer program storage medium comprising a program according to claim 8.

10. A device for spatially locating and tracking a medical instrument (8) and a patient (5) or part of a patient's body, comprising a computer (10) and a single camera array (1) which comprises at least two cameras (2) and is connected to the computer (10), wherein the camera array (1) is configured to record tracking markings (4) on the medical instrument (8) and the patient (5) or part of the patient's body, wherein the computer (10) is configured by means of a program loaded onto it to extract the tracking markings from the camera recordings and track them, in order to locate and track the medical instrument (8) and the patient (5) or part of the body, wherein the camera array (1) is also configured to produce video recordings of the surface of the patient (5) or part of the body, wherein the computer is also configured by means of the program to identify surface portions in the video recordings by means of a morphing method and to correlate them by means of a matching method with corresponding surface portions in the stored patient data set, in order to assign the spatial position of the patient (5) or part of the body of the patient (5) and that of the patient data set.

11. The device according to claim 10, wherein both cameras (2) are configured as video cameras for video recordings in the visible light range.

12. The device according to claim 10 or 11, wherein both cameras (2) are configured as hybrid cameras for video recordings in the visible light range and in the infrared range.

13. The device according to any one of claims 10 to 12, wherein the camera array (1) is configured to be fixed or movable.

14. The device according to any one of claims 10 to 13, wherein the device comprises a data output device (13) such as a screen for outputting the stored patient data set and/or the patient (5) or part of the patient's body and/or the medical instrument (8) and/or comprises a data input device (12), in particular a keyboard, an x-ray device, an ultrasound tomograph, a computer tomograph, a magnetic resonance tomograph, a positron emission tomograph (PET) or a single-photon emission tomography (SPET) tomograph, by means of which data on the medical instrument (8) and/or the patient (5) or part of the patient's body can be obtained.

15. The device according to any one of claims 10 to 14, wherein the device comprises a database (11) in which characteristic patient data sets can be stored as reference data sets or into which patient data sets can be read as reference data sets, in particular by means of the data input device (12), and stored.

16. The device according to any one of claims 10 to 15, wherein the device is configured in an instrument such as a microscope or an endoscope.

## Revendications

1. Procédé de repérage et d'enregistrement pour localiser et suivre dans l'espace un instrument médical (8) et un patient (5) ou une partie corporelle d'un patient, ayant un système de caméras (1) comportant au moins deux caméras (2), ainsi que pour associer mutuellement la position spatiale du patient (5) ou de la partie corporelle du patient (5) et un ensemble de données de patient acquis et mémorisé, comportant les étapes consistant à :
- enregistrer des marqueurs de repérage (4) sur l'instrument médical (8) et le patient (5) ou la partie corporelle du patient, les extraire des enregistrements de caméras et les suivre afin de localiser et de suivre l'instrument médical (8) et le patient (5) ou la partie corporelle, et
- créer des enregistrements vidéo de la surface du patient (5) ou de la partie corporelle, dans lesquels des portions de surface sont identifiées par un procédé de morphisme assisté par ordinateur, lequel procédé établit une correspondance avec des portions de surface correspondantes dans l'ensemble de données de patient mémorisé par un procédé de mise en correspondance assisté par ordinateur, afin d'associer la position spatiale du patient (5) ou de la partie corporelle d'un patient (5) et l'ensemble de données du patient,
au moyen de ce même système de caméras (1).

2. Procédé selon la revendication 1, dans lequel des points, des ensembles de points, des tâches ponctuelles ou des contours de la surface sont utilisés comme portions de surface identifiées.

3. Procédé selon l'une quelconque des revendications 1 à 2, dans lequel l'ensemble de données de patient mémorisé est mis en correspondance avec les portions de surface au moyen d'un procédé de photoconsistance.

4. Procédé selon l'une quelconque des revendications 1 à 3, dans lequel l'ensemble de données de patient mémorisé est mis en correspondance avec les portions de surface au moyen d'un procédé de corrélation de points.

5. Procédé selon l'une quelconque des revendications 1 à 4, dans lequel la mémorisation de l'ensemble de données de patient et des portions de surface identifiées, ainsi que l'établissement de la correspondance sont effectués avec l'aide d'un ordinateur au moyen d'un système de navigation médicale, auquel le système de caméras (1) est associé.

6. Procédé selon l'une quelconque des revendications 1 à 5, dans lequel les caméras pour la localisation, l'association de la position spatiale et de l'ensemble de données de patient génèrent des enregistrements vidéo dans le domaine de la lumière visible.

7. Procédé selon l'une quelconque des revendications 1 à 6, dans lequel les caméras pour la localisation et le suivi génèrent des enregistrements dans le domaine de la lumière infrarouge et les caméras pour l'association de la position spatiale et de l'ensemble de données de patient enregistrent des enregistrements vidéo dans le domaine de la lumière visible.

8. Programme qui, lorsqu'il s'exécute sur un ordinateur ou lorsqu'il est chargé dans un ordinateur, amène l'ordinateur à mettre en oeuvre un procédé selon l'une quelconque des revendications 1 à 7.

9. Support de mémorisation de programme informatique comportant un programme selon la revendication 8.

10. Dispositif de localisation et de suivi spatial d'un instrument médical (8) et d'un patient (5) ou d'une partie corporelle d'un patient, lequel dispositif comporte un ordinateur (10) et un seul système de caméras (2) relié à l'ordinateur (10) et ayant au moins deux caméras (2), dans lequel le système de caméras (1) est configuré pour enregistrer des marqueurs de repérage sur l'instrument médical (8) et le patient (5) ou la partie corporelle du patient, dans lequel l'ordinateur (10), au moyen d'un programme chargé sur celui-ci, est conçu pour extraire des enregistrements de caméras et suivre les marqueurs de repérage afin de localiser et de suivre l'instrument médical (8) et le patient (5) ou la partie corporelle, dans lequel le système de caméras (1) est de plus conçu pour générer des enregistrements vidéo de la surface du patient (5) ou de la partie corporelle, dans lequel l'ordinateur (10), au moyen du programme, est de plus conçu pour identifier des portions de surface dans les enregistrements vidéo au moyen d'un procédé de morphisme et pour établir une correspondance avec des portions de surface correspondantes dans l'ensemble de données de patient mémorisé au moyen d'un procédé de mise en correspondance, afin d'associer la position spatiale du patient (5) ou la partie corporelle du patient (5) et l'ensemble de données du patient.

11. Dispositif selon la revendication 10, dans lequel les deux caméras (2) sont conçues comme des caméras vidéo pour un enregistrement vidéo dans le domaine de la lumière visible.

12. Dispositif selon la revendication 10 ou 11, dans lequel les deux caméras (2) sont conçues comme des caméras hybrides pour un enregistrement vidéo dans le domaine de la lumière visible et dans le domaine de l'infrarouge.

13. Dispositif selon l'une quelconque des revendications 10 à 12, dans lequel le système de caméras (1) est conçu de manière fixe ou mobile.

14. Dispositif selon l'une quelconque des revendications 10 à 13, dans lequel le dispositif inclut un dispositif de sortie de données (13), tel qu'un écran pour la sortie de l'ensemble de données de patient mémorisé et/ou du patient (5) ou de la partie corporelle du patient et/ou de l'instrument médical (8), et/ou un dispositif d'entrée de données (12), en particulier un clavier, un dispositif de radiographie, un échotomographe, un tomographe assisté par ordinateur, un tomographe à résonance magnétique, un tomographe à émission de positrons (PET) ou un tomographe à émission de photons uniques (SPET), au moyen desquels des données concernant l'instrument médical (8) et/ou le patient (5) ou la partie corporelle du patient peuvent être acquises.

15. Dispositif selon l'une quelconque des revendications 10 à 14, dans lequel le dispositif inclut une base de données (11) dans laquelle des ensembles de données de patient caractéristiques peuvent être mémorisés en tant qu'ensembles de données de référence, ou dans lequel des ensembles de données de patient, en particulier au moyen du dispositif d'entrée de données (12), peuvent être lus et mémorisés en tant qu'ensembles de données de référence.

16. Dispositif selon l'une quelconque des revendications 10 à 15, dans lequel le dispositif est formé dans un instrument, tel qu'un microscope ou un endoscope.
